# EUROPEAN PATENT APPLICATION

(11) **EP 4 796 544 A1**
(43) Date of publication of application: **26.08.2026**
(21) Application number: 23956168.1
(22) Date of filing: 16.10.2023
(51) Int. Cl.: C07D 487/04, A61K 31/519, A61P 35/00

(54) **CRYSTALLINE FORM OF TRIAZOLOPYRIMIDINONE DERIVATIVE**

(71) Applicant: ST Pharm Co., Ltd., Siheung-si, Gyeonggi-do 15086 (KR)
(72) Inventor: KIM, Uk-Il, Ansan-si, Gyeonggi-do 15610 (KR); BANG, Hyung Tae, Ansan-si, Gyeonggi-do 15610 (KR)
(74) Representative: dompatent
(86) International application number: PCT/KR2023/015953
(87) International publication number: WO 2025/084446

(57) **Abstract**

The present invention relates to a polymorphism of a triazolopyrimidinone derivative exhibiting tankyrase inhibitory activity, and a preparation method therefor. The novel polymorphism of the present invention exhibits excellent non-hygroscopicity and stability, and exhibits excellent solubility and bioavailability, and thus is suitable as a raw material for pharmaceutical preparations.

## Description

### [Technical Field]

The present invention relates to a crystalline form of a triazolopyrimidinone derivative exhibiting tankyrase inhibitory activity. The present invention also relates to a method for preparing the crystalline form of the triazolopyrimidinone derivative.

### [Background Art]

Tankyrase belongs to the poly(ADP-ribose) polymerase (PARP) protein family which consists of 17 members sharing a catalytic PARP domain. Recently, it has been reported that intracellular axin levels are affected by PARP enzyme family members, tankyrase-1 and tankyrase-2 (also known as PARP5a and PARP5b, respectively) (Huang et al., 2009, Nature, 461 (7264): 614-620).

Inhibitors of tankyrase-1 and tankyrase-2 are known to be used for the treatment of various solid tumors such as colorectal carcinoma, colon cancer, gastric cancer, hepatocellular carcinoma, breast cancer, medulloblastoma, melanoma, non-small cell lung cancer, pancreatic adenocarcinoma, and prostate cancer. In addition, the inhibitors of tankyrase-1 and tankyrase-2 have therapeutic potential for other diseases in addition to the cancer diseases, including osteoporosis, osteoarthritis, polycystic kidney disease, pulmonary fibrosis, diabetes, schizophrenia, vascular disease, cardiac disease, non-oncogenic proliferative disease, and neurodegenerative diseases such as Alzheimer's disease.

As described above, the demand for novel therapeutic agents capable of being used for cancer and hyperproliferative conditions continues, and efforts are being made to develop novel pharmaceutical compounds capable of selectively inhibiting tankyrase enzymes. In particular, triazolopyrimidinone derivatives of the following Chemical Formula I are known as selective tankyrase inhibitors (International Patent Publication No. WO2016/006974), and are being developed as therapeutic agents for colorectal cancer in patients with a colorectal cancer-inducing gene (KRAS: Kirsten rat sarcoma virus) mutant genotype or Erbitux non-responsive patients (European Journal of Cancer, 173, (2022), 41-51).

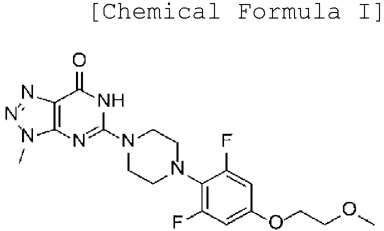

In the pharmaceutical manufacturing process, the physical state of the pharmaceutical raw material, i.e., whether crystalline or amorphous, is crucial in designing an appropriate dosage form. However, crystalline raw materials have low solubility, which may result in reduced bioavailability per unit weight. In contrast, amorphous forms are unstable and may present challenges in controlling drug release and blood concentration levels.

Therefore, it is necessary to develop a novel crystalline form of the triazolopyrimidinone derivative of Chemical Formula I that overcomes these limitations while exhibiting improved physicochemical properties.

### [Disclosure]

### [Technical Problem]

An object of the present invention is to provide a crystalline form of a triazolopyrimidinone derivative having excellent non-hygroscopicity and stability, and a preparation method thereof.

Another object of the present invention is to provide a crystalline form of a triazolopyrimidinone derivative capable of improving solubility and bioavailability, and a preparation method thereof.

### [Technical Solution]

In order to achieve the above purpose, the present inventors conducted research and made efforts to prepare crystalline forms A and B described below, and confirmed that all of these crystalline forms exhibited excellent effects, thereby completing the present disclosure.

### Crystalline Form A and Preparation Method Thereof

The present invention provides a crystalline form A of a compound represented by the following Chemical Formula I, which has a powder X-ray diffraction pattern comprising peaks at diffraction angles (2θ ± 0.2°) of 6.31°, 8.72°, 10.92°, 12.12°, 13.16°, and 15.98°:

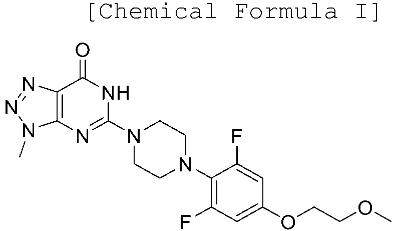

According to an embodiment of the present invention, the powder X-ray diffraction pattern of the crystalline form A may further comprise at least one peak at a diffraction angle (2θ ± 0.2°) of 5.43°, 7.93°, 17.56°, or 18.22°.

According to an embodiment of the present invention, the powder X-ray diffraction pattern of the crystalline form A may further comprise at least one peak at a diffraction angle (2θ ± 0.2°) of 20.63°, 21.14°, 22.23°, 23.13°, 23.98°, 25.31°, 26.08°, 26.99°, or 32.04°.

According to an embodiment of the present invention, the crystalline form A may have a melting point of 235 to 245 °C when the heating rate is 2 °C/min, and specifically may have a melting point of about 238 to about 242 °C. More specifically, the crystalline form A may have a melting point of approximately 239.7 °C when the heating rate is 2 °C/min.

In addition, the present invention provides a method for preparing the crystalline form A. Specifically, the above preparation method may comprise the following steps:
(A-1) adding a mixed solvent of acetone and water to a compound represented by the following Chemical Formula I, followed by stirring; and
(A-2) filtering and drying the resulting solid.

According to an embodiment of the present invention, a volume ratio of acetone and water in the mixed solvent may be 4 : 6 to 6 : 4, and preferably 5 : 5.

### Crystalline Form B and Preparation Method Thereof

The present invention provides a crystalline form B of the compound represented by Chemical Formula I above, which has a powder X-ray diffraction pattern comprising peaks at diffraction angles (2θ ± 0.2°) of 7.83°, 9.47°, 10.24°, 12.32°, 15.16°, and 16.29°.

According to an embodiment of the present invention, the powder X-ray diffraction pattern of the crystalline form B may further comprise at least one peak at a diffraction angle (2θ ± 0.2°) of 6.12°, 18.46°, or 19.05°.

According to an embodiment of the present invention, the powder X-ray diffraction pattern of the crystalline form B may further comprise at least one peak at a diffraction angle (2θ ± 0.2°) of 20.63°, 21.33°, 24.39°, 25.19°, 26.66°, or 27.54°.

According to an embodiment of the present invention, the crystalline form B may have a melting point of approximately 245 to 255 °C when the heating rate is 2 °C/min, and specifically may have a melting point of approximately 247 to about 250 °C. More specifically, the crystalline form B may have a melting point of approximately 248.7 °C when the heating rate is 2 °C/min.

Further, the present invention provides a method for preparing the crystalline form B. Specifically, the above preparation method may comprise the following steps:
(B-1) adding acetone, acetonitrile, 2-butanone, toluene, or isopropanol to the compound represented by Chemical Formula I above, followed by stirring; and
(B-2) filtering and drying the resulting solid.

### [Advantageous Effects]

The crystalline form of the present invention may have low hygroscopicity and excellent stability under accelerated and long-term storage conditions, and may be stably maintained without changes in amount over a long period of time, which is advantageous for pharmaceutical preparations.

Further, the crystalline form of the present invention may have high solubility and bioavailability, and thus may be usefully employed as a pharmaceutical raw material.

### [Description of Drawings]

FIG. 1 shows the powder X-ray diffraction (PXRD) results of crystalline form A according to Example 1 of the present invention.
FIG. 2 shows the powder X-ray diffraction (PXRD) results of crystalline form B according to Example 2 of the present invention.

### [Best Mode]

Hereinafter, the present invention will be described in more detail through Examples and Experimental Examples to facilitate understanding. However, the following Examples and Experimental Examples are only provided to illustrate the present invention, and the content of the present invention is not limited to these Examples. These Examples and Experimental Examples of the present invention are provided to more comprehensively illustrate the invention to a person of ordinary skill in the art.

The reagents and solvents described below were purchased from Sigma-Aldrich Korea, TCI, and Daejung Chemicals & Metals unless otherwise specified, and HPLC was measured using the 1000 series of Agilent Technologies, Inc.

In addition, the various solid forms of 5-(4-(2,6-difluoro-4-(2-methoxyethoxy)phenyl)piperazin-1-yl)-3-methyl-3,6-dihydro-7H-[1,2,3]triazolo[4,5-d]pyrimidin-7-one prepared by the following examples were identified by X-ray diffraction patterns using X-ray diffraction analysis and measurement of melting points.

Specifically, the powder X-ray diffraction analysis was conducted using a Bruker D8 Focus diffractometer equipped with a rotational meter and a solid-state detector, with measurements performed under the conditions of an analysis range of 5° to 50°, Step of 0.020°, and Step time of 171.6 seconds using Cu-Kα rays. In addition, the measurement of melting points is performed at 200 to 260 °C and a heating rate of 2 °C/min, using a Buchi M-565 Melting Point instrument.

### Preparation Example. Preparation of 5-(4-(2,6-difluoro-4-(2-methoxyethoxy)phenyl)piperazin-1-yl)-3-methyl-3,6-dihydro-7H-[1,2,3]triazolo[4,5-d]pyrimidin-7-one

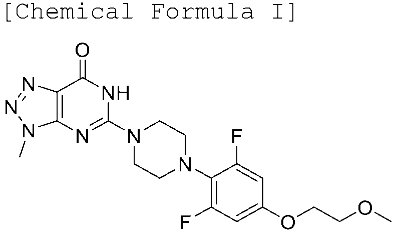

To 5-(4-(2,6-difluoro-4-(2-methoxyethoxy)phenyl)piperazin-1-yl)-7-isopropoxy-3-methyl-3H-[1,2,3]triazolo[4,5-d]pyrimidine (15 g, 32.4 mmol), 92 mL of acetic acid and 16.1 mL of sulfuric acid were added, and the mixture was heated to an internal temperature of 45 to 50 °C and stirred for 3 hours. After the reaction was complete, the reaction solution was cooled to room temperature, and 230 mL of purified water was added dropwise over 30 minutes, with stirring. After the addition was complete, the reaction mixture was cooled to an internal temperature of 5 to 10 °C and stirred for 1 hour. The precipitated crystals were filtered and diluted in 150 mL of acetone. Then, the mixture was heated to an internal temperature of 40 to 45 °C and stirred for 1 hour. The reaction mixture was slowly cooled to 5 to 10 °C, and 150 mL of purified water was added. The precipitated crystals were stirred for 30 minutes, filtered, and dried under reduced pressure to obtain the title compound (11.5 g, 84%) as an off-white solid.

### Example 1. Preparation of Crystalline Form A

The 5-(4-(2,6-difluoro-4-(2-methoxyethoxy)phenyl)piperazin-1-yl)-3-methyl-3,6-dihydro-7H-[1,2,3]triazolo[4,5-d]pyrimidin-7-one (1 g) prepared in Preparation Example above of the present invention, was added to acetone : water (50 : 50) and stirred overnight at 40 to 50 °C. The resulting solid was filtered and dried in a convection oven at 50 °C to obtain crystalline form A.

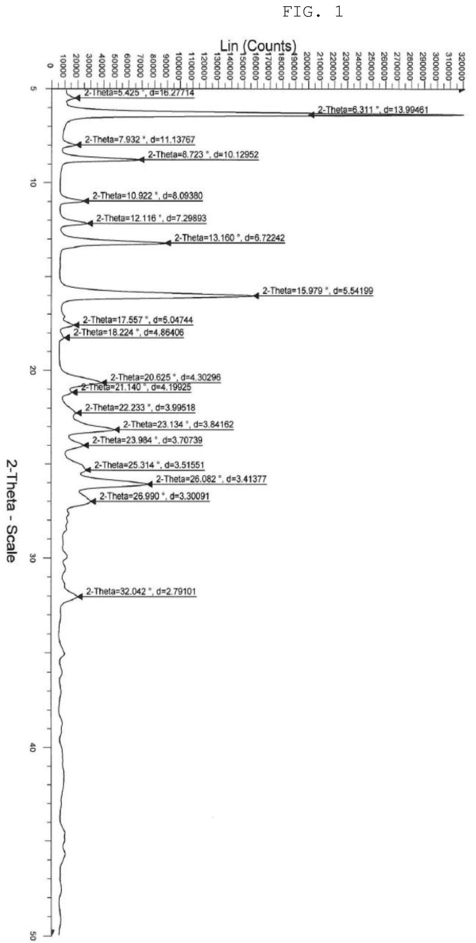

The powder X-ray diffraction (PXRD) peaks and melting point of the obtained solid were analyzed, and the PXRD results are shown in FIG. 1.

As confirmed in FIG. 1, the solid compound exhibited main peaks at 2θ (±0.2°) values of 6.31°, 8.72°, 10.92°, 12.12°, 13.16°, and 15.98°.

In addition, it was confirmed that the solid compound had a melting point of 239.7 °C when the heating rate was 2 °C/min.

### Example 2. Preparation of Crystalline Form B

The 5-(4-(2,6-difluoro-4-(2-methoxyethoxy)phenyl)piperazin-1-yl)-3-methyl-3,6-dihydro-7H-[1,2,3]triazolo[4,5-d]pyrimidin-7-one (1 g) prepared in Preparation Example above of the present invention, was added to various solvents according to Table 1 below, and stirred overnight at 40 to 50 °C. The resulting solid was filtered and dried in a convection oven at 50 °C to obtain crystalline form B.

**[Table 1]**

| Entry | Solvent | Purity | Crystalline Form |
|---|---|---|---|
| 2-1 | Acetone (10V) | 98.7% -> 99.20% | |
| 2-2 | Acetonitrile (10V) | 98.7% -> 99.18% | |
| 2-3 | 2-Butanone (10V) | 98.7% -> 99.19% | Crystalline B |
| 2-4 | Toluene (10V) | 98.7% -> 99.23% | |
| 2-5 | Isopropanol (10V) | 98.7% -> 98.90% | |

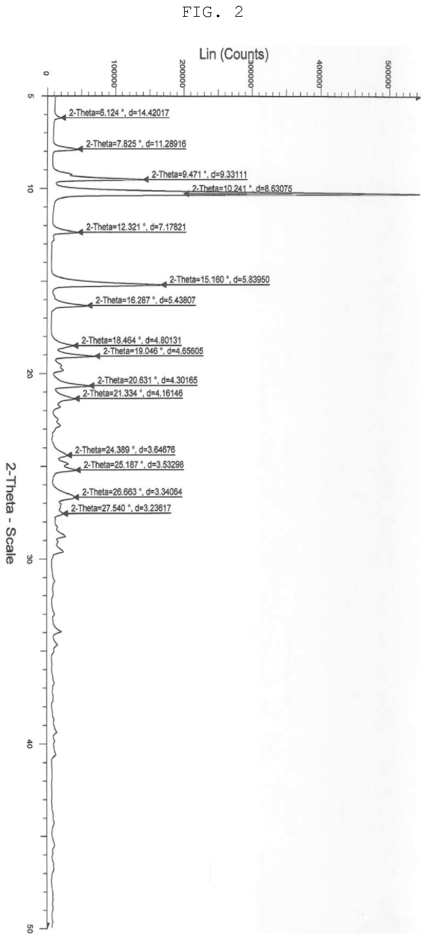

The powder X-ray diffraction (PXRD) peaks and melting point of the obtained solid were analyzed, and the PXRD results are shown in FIG. 2.

As confirmed in FIG. 2, the solid compound exhibited main peaks at 2θ (±0.2°) values of 7.83°, 9.47°, 10.24°, 12.32°, 15.16°, and 16.29°.

In addition, it was confirmed that the solid compound had a melting point of 248.7 °C when the heating rate was 2 °C/min.

### Experimental Example 1. Hygroscopicity (DVS) Measurement Experiment

The crystalline forms prepared in Examples of the present invention were tested for hygroscopicity.

### a. Equipment Used in the Experiment

- Model Name: DVS Intrinsic (Surface Measurement Systems)
- Specifications
   Temperature Range: 20 - 40 °C
   Temperature stability: ± 0.2 °C
   Humidity range: 0 - 98% RH
   RH Accuracy: ±1% RH
   Typical gas (Nitrogen + water vapor) flow rate: 200 sccm
   Sample chamber: 40 mm wide x 50 mm deep x 50 mm high
   Heating system: Peltier + Cartridge

### b. Sample Information

Experiments were performed on the crystalline forms of Examples above.

### c. Experimental Conditions

- Temperature: 25 °C
- Humidity range: Starting from 0%, reaching up to 95%
- Solvent: Water

### d. Experimental Method

A predetermined amount (10 to 20 mg) of the crystalline form prepared in each Example was placed on the sample pan in the sample chamber, and then nitrogen gas was purged through the tube connected to the sample chamber for an initial hour to remove moisture from both the chamber and the sample. The humidity in the chamber was controlled by adjusting the water vapor, and the target relative humidity was increased from 0% to about 10% step by step to measure the moisture content of the crystalline forms. The mass of each sample was measured (from 0% RH to 95% RH) and the resulting changes in sample weight with varying humidity levels were plotted as a graph.

The change in moisture content of the crystalline forms according to Examples is as shown in Table 2 below.

**[Table 2]**

| Relative Humidity (%) | Crystalline Form B (% Change in Weight) |
|---|---|
| 0 | 0 |
| 10 | 0.00 |
| 20 | 0.01 |
| 30 | 0.01 |
| 40 | 0.02 |
| 50 | 0.03 |
| 60 | 0.05 |
| 70 | 0.06 |
| 80 | 0.08 |
| 90 | 0.12 |
| 95 | 0.18 |

As a result of the analysis, as shown in Table 2 above, the crystalline forms of the present invention were observed to have excellent non-hygroscopicity with almost no change in moisture content. This indicates that the crystalline form of the present invention is superior in formulation during the entire process of manufacturing, storage, and formulation of the active ingredient.

### Experimental Example 2. Accelerated Stability Test

The powder of crystalline form according to each Example of the present invention was double-packaged in a PE bag and triple-packaged in an Aluminum bag, and left at 40 ± 2 °C, RH 75 ± 5% for 0 and 6 months, and then the impurities were measured using HPLC. The results are shown in Table 3 below.

**[Table 3]**

| Test Items | Example 1 | | Example 2 | |
|---|---|---|---|---|
| | 0 month (initial) | 6 month | 0 month (initial) | 6 month |
| Related Substances (%) | 0.32 | 0.37 | 0.10 | 0.09 |
| Amount (%) | 98 | 98 | 102 | 100 |
| Crystalline Form | Type A | Type A | Type B | Type B |

It was confirmed from the results in Table 3 above that the crystalline forms of the present invention remained crystalline, with no significant changes in related substances and amount observed. Therefore, it was appreciated that the crystalline forms of the present invention were able to stably maintain high purity for 6 months under accelerated conditions, showing excellent stability, little change in moisture content, and significantly lower hygroscopicity.

### Experimental Example 3. Long-Term Stability Test

The powder of crystalline form according to each Example of the present invention was double-packaged in a PE bag and triple-packaged in an Aluminum bag, and left at 25 ± 2 °C, RH 60 ± 5% for 0 and 12 months, and then the impurities were measured using HPLC. The results are shown in Table 4 below.

**[Table 4]**

| Test Items | Example 1 | | Example 2 | |
|---|---|---|---|---|
| | 0 month (initial) | 12 month | 0 month (initial) | 12 month |
| Related Substances (%) | 0.32 | 0.33 | 0.10 | 0.10 |
| Amount (%) | 98 | 99 | 102 | 100 |
| Crystalline Form | Type A | Type A | Type B | Type B |

It was confirmed from the results in Table 4 above that the crystalline forms of the present invention remained crystalline, with no significant changes in related substances and amount observed. Therefore, it was appreciated that the crystalline forms of the present invention were able to stably maintain high purity for 12 months, showing excellent stability, little change in moisture content, and significantly lower hygroscopicity.

## Claims

1. A crystalline form A of a compound represented by the following Chemical Formula I, which has a powder X-ray diffraction pattern comprising peaks at diffraction angles (2θ ± 0.2°) of 6.31°, 8.72°, 10.92°, 12.12°, 13.16°, and 15.98°:

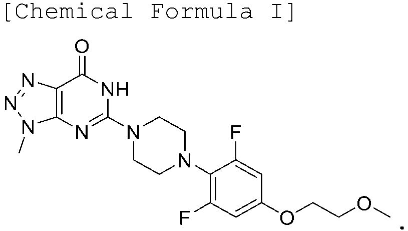

2. The crystalline form A of claim 1, wherein the powder X-ray diffraction pattern further comprises at least one peak at a diffraction angle (2θ ± 0.2°) of 5.43°, 7.93°, 17.56°, or 18.22°.

3. The crystalline form A of claim 2, wherein the powder X-ray diffraction pattern further comprises at least one peak at a diffraction angle (2θ ± 0.2°) of 20.63°, 21.14°, 22.23°, 23.13°, 23.98°, 25.31°, 26.08°, 26.99°, or 32.04°.

4. The crystalline form A of claim 1, wherein the crystalline form A has a melting point of 235 to 245 °C when the heating rate is 2 °C/min.

5. A method for preparing the crystalline form A of the compound represented by Chemical Formula I according to claims 1 to 4, comprising:
(A-1) adding a mixed solvent of acetone and water to a compound represented by the following Chemical Formula I, followed by stirring; and
(A-2) filtering and drying the resulting solid:

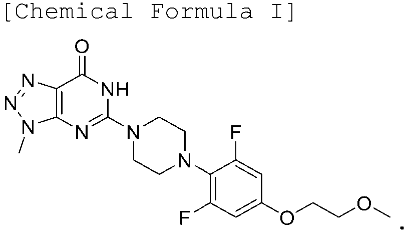

6. The method of claim 5, wherein a volume ratio of acetone and water in the mixed solvent is 4 : 6 to 6 : 4.

7. A crystalline form B of a compound represented by the following Chemical Formula I, which has a powder X-ray diffraction pattern comprising peaks at diffraction angles (2θ ± 0.2°) of 7.83°, 9.47°, 10.24°, 12.32°, 15.16°, and 16.29°:

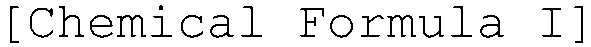

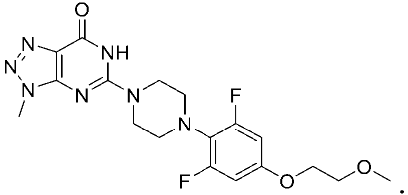

8. The crystalline form B of claim 7, wherein the powder X-ray diffraction pattern further comprises at least one peak at a diffraction angle (28 ± 0.2°) of 6.12°, 18.46°, or 19.05°.

9. The crystalline form B of claim 8, wherein the powder X-ray diffraction pattern further comprises at least one peak at a diffraction angle (2θ ± 0.2°) of 20.63°, 21.33°, 24.39°, 25.19°, 26.66°, or 27.54°.

10. The crystalline form B of claim 7, wherein the crystalline form B has a melting point of 245 to 255 °C when the heating rate is 2 °C/min.

11. A method for preparing the crystalline form B of the compound represented by Chemical Formula I according to claims 7 to 10, comprising:
(B-1) adding acetone, acetonitrile, 2-butanone, toluene, or isopropanol to a compound represented by the following Chemical Formula I, followed by stirring; and
(B-2) filtering and drying the resulting solid:

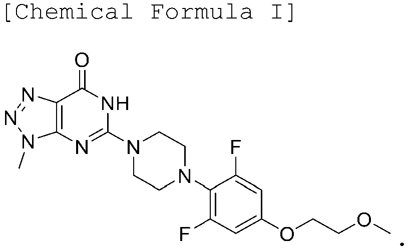

12. A pharmaceutical composition for use in preventing or treating cancer, comprising the crystalline form A according to claims 1 to 4 and the crystalline form B according to claims 7 to 10.
